# EUROPEAN PATENT APPLICATION

(11) **EP 4 559 571 A1**
(43) Date of publication of application: **28.05.2025**
(21) Application number: 24201420.7
(22) Date of filing: 19.09.2024
(51) Int. Cl.: B01J 20/26, B01J 20/28

(54) **LOW RESIDUE SAMPLING MATERIAL FOR SMALL MOLECULE DRUG DETECTION AND USE OF THE SAME**

(30) Priority: 22.11.2023 CN 202311566175
(71) Applicant: Zhejiang Orient Gene Biotech Co., Ltd, Anji Huzhou Zhejiang 313300 (CN)
(72) Inventor: SHEN, Lili, Huzhou (CN); FANG, Jianqiu, Huzhou (CN)
(74) Representative: Metida

(57) **Abstract**

A low residue sampling material for a small molecule drug detection, and a use of the low residue sampling material in the small molecule drug detection are provided in the present invention. The low residue sampling material for small molecule drug detection includes a continuous network skeleton structure, a density is in a range of from 8.3 kg/m³ to 9.3 kg/m³, an opening rate is greater than 99%, and a skeleton diameter is in a range of from 5 µm to 10 µm; to effectively solve a problem of low sampling effectiveness during a sampling and transfer process before detecting small molecule drugs, and thereby affecting an accuracy of detection in the prior art.

## Description

### TECHNICAL FIELD

The present invention relates to a small molecule drug detection technology field, and in particular to a low residue sampling material for a small molecule drug detection, and a use of the low residue sampling material in the small molecule drug detection.

### BACKGROUND

At present, body fluid samples are usually collected using water-absorbent materials (absorbent elements), the water-absorbent materials are dry at first, and liquid samples or fluid samples can be absorbed through capillaries or other properties of materials of the absorbent elements, thereby keeping the fluid samples in the absorption elements. Absorbent materials can be any materials that can absorb liquid, such as sponge, filter paper, polyester fiber, gel, non-woven fabric, cotton, polyester film, yarn, flocking and the like. Porous materials are used for collection, such as common sponges or filter papers. Absorbed samples are squeezed and released from the absorbent elements, and released samples can be directly used to detect whether the sample includes an interested analyte in the sample. Alternatively, porous films with the liquid samples are immersed in a treatment liquid, and the liquid samples on the porous films can be eluted into the treatment liquid, thus the interested analyte in the sample can be better detected in a detection device.

However, some of the effective substances to be tested are remained in a sampling tube due to adsorption problems, an effectiveness of sampling is low, and cannot be fully released for subsequent testing. Although a series of extrusion devices are developed in prior art, an adsorption of small molecular drugs, such as small molecular drug components BUP, 6MAM, PCP, FEN, BZO and THC (PARENT and METABOLITE), by adsorption materials (such as porous materials) in the sampling tube is not solved. Among them, a known THC adsorption is the most serious adsorption, and a recovery rate is about less than 20%. Due to above reasons, saliva samples collected using sampling materials usually have inaccurate results when tested, because active ingredients in the saliva samples are not fully utilized and detected. The phenomenon has a great influence on an accuracy of a detection.

### SUMMARY

A purpose of the present invention is to provide a low residue sampling material for small molecule drug detection, to solve a problem of low sampling effectiveness during a sampling and transfer process before detecting small molecule drugs, and thereby affecting an accuracy of detection in the prior art. In particular, the present invention provides a use of the sampling material in small molecule drug sampling for detection.

The present invention provides the following technical solution: A low residue sampling material for small molecule drug detection, including a continuous network skeleton structure, a density is in a range of from 8.3 kg/m³ to 9.3 kg/m³, an opening rate is greater than 99%, and a skeleton diameter is in a range of from 5 µm to 10 µm.

The small molecule drugs described of the present invention refer to molecules with a molecular weight below 1000 Daltons (Da), in particular, molecules with molecular weight in a range of from 237 to 468. The small molecule drugs include but not limited to drug abuse small molecules, for example, BUP (buprenorphine, 467.64), 6-MAM (6-monoacetylmorphine, 327.37), PCP (ketamine, 237.73), FEN (fentanyl, 336.46), BZO (benzodiazepine, 284.74), THC (tetrahydrocannabinol,314.462, including PARENT and METABOLITE).

The continuous network skeleton structure of the present invention has interconnected skeleton nodes, thereby forming a network structure and providing sufficient adsorption performance for the sampling material. At the same time, the effective opening rate and skeleton diameter ensure that samples can be effectively extruded, and residues are reduced, thereby making a recovery rate of sample detection high.

In one embodiment, materials with the continuous network skeleton structure include polyurethane foam, melamine foam, activated carbon, polydimethylsiloxane sponge, modified silica gel, polyester fibers and polyester films. The melamine foam is a foam-like material composed of melamine formaldehyde condensate.

In some embodiments of the present invention, a method for preparing the melamine foam includes the following steps: impregnating the melamine foam by a hydrophobic modification emulsion; and then, performing an emulsion polymerization under a heating condition, and in situ growing nanospheres on a melamine foam skeleton; finally, washing and drying to obtain superhydrophobic modified melamine foam. The hydrophobic modification emulsion includes a modified monomer, an initiator, a crosslinker, an emulsifier, a low surface energy modifier, a solvent and water. On one hand, a large porosity of the continuous network skeleton structure can provide sufficient capillary force to fully absorb detection samples. On the other hand, through an hydrophobic modification of the melamine foam, the melamine foam can release the samples more effectively under extrusion, further reducing the residual.

A method for preparing the hydrophobic modification emulsion includes the following steps: dispersing the initiator in deionized water, and then adding the modified monomer, the crosslinker, the emulsifier and the low surface energy modifier, to obtain a mixed solution; and dispersing the mixed solution in the solvent to obtain the hydrophobic modification emulsion. By a weight part, in the hydrophobic modified emulsion, the modified monomer is in a range of from 21 parts to 30 parts, the initiator is 1 part, the crosslinker is in a range of from 20 parts to 22 parts, the emulsifier is in a range of from 10 parts to 16 parts, and the low surface energy modifier is in a range of from 4.3 parts to 8 parts.

The modified monomer can be one of styrene, acrylic acid and methyl methacrylate; and the low surface energy modifier can be stearic acid or dimethicone.

The initiator can be sodium persulfate or ammonium persulfate; and the crosslinker can be one of divinylbenzene, N,N-methylenebisacrylamide, trimethylolpropane triacrylate, ethylene glycol dimethacrylate and dipentaerythritol pentaacrylate.

The emulsifier can be one of Span80, Tween80, hexadecyltrimethylammonium bromide, sodium dodecyl sulfate and ethylene glycol fatty acid ester.

The solvent can be one of anhydrous ethanol, tetrahydrofuran, N,N-dimethylformamide, dimethyl sulfoxide, dioxane and N-methylpyrrolidone.

A step of impregnating the melamine foam can be ultrasonic soaking the melamine foam in the hydrophobic modification emulsion for 10 min to 60 min; the emulsion polymerization can be carried out at 40°C to 80 °C for 4 hours to 24 hours; and the drying can be air blast drying at 40°C to 100°C for 8 hours to 24 hours.

In one embodiment, a method for preparing the modified silica gel includes the following steps:
step (1), adding pyridine and 2-methyl-1-butanol into a three-necked flask, and then placing the three-necked flask in an ice water bath; after cooling, adding p-toluenesulfonyl chloride, and controlling a temperature of the three-necked flask and stirring, and then storing the three-necked flask in a refrigerator; removing the three-necked flask and placing the three-necked flask in the ice water bath, and adding distilled water and stirring; after a reaction is completed, adding ether for extraction, collecting organic phases and washing with hydrochloric acid and water; and then adding anhydrous sodium sulfate for drying, and filtering to obtain a polymer 1;
step (2), adding 4-(4-hydroxyphenyl) azobenzoic acid, sodium bicarbonate and dimethylacetamide into a three-necked flask, heating and stirring until solids are completely dissolved, and then decreasing temperature; adding the polymer 1 into the three-necked flask, increasing temperature and stirring; and after stirring, cooling, adding distilled water and suction filtering to obtain a polymer 2; and adding the polymer 2, potassium carbonate powder and potassium iodide into a three-necked flask in sequence, and then adding dimethyl methacrylate to obtain a reaction solution; adding 6-chloro-1-hexanol dropwise into the reaction solution under stirring, heating and reacting; and after reaction is completed, cooling, adding distilled water to the three-port flask, pumping and suction filtering to obtain a polymer 3;
step (3), adding the polymer 3, dichloromethane, 4-dimethylaminopyridine and methacrylic acid into a single flask, stirring until completely dissolved; adding a mixed solution of dicyclohexylcarbodiimide and dichloromethane, increasing temperature and reacting; and after a reaction is completed, filtrating, and rotary evaporating to obtain a polymer 4; and dissolving the polymer 4 in an hydrofluoric acid solution, adding hydrogen peroxide, heating, stirring and reacting; and after a reaction is completed, heating and suction filtering to obtain a fluorine-containing chiral side chain azobenzene monomer;
step (4), dissolving the fluorine-containing chiral side chain azobenzene monomer in cyclopentanone, stirring until completely dissolved to obtain a mixed solution, and then adding the mixed solution into a liquid silica gel, stirring to mix evenly, and vacuum freeze-drying, to obtain the sampling material.

In the above process of the method for preparing the modified silica gel, a synthesis process of the fluorine-containing chiral side chain azobenzene monomer is as follows:

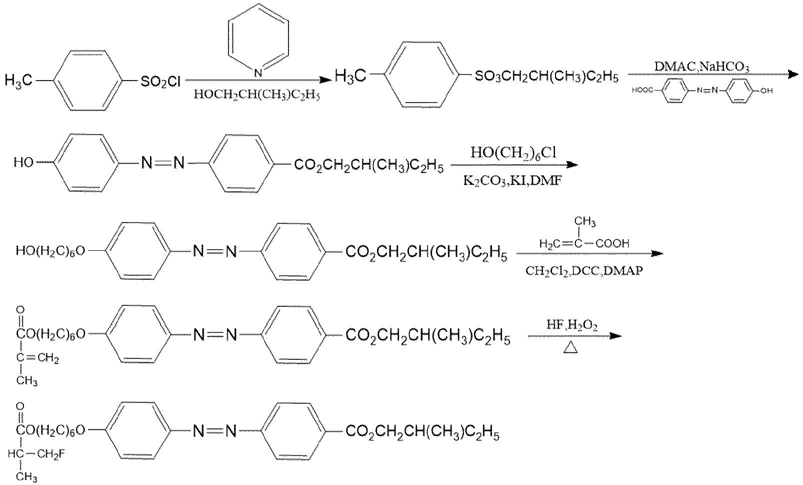

The fluorine-containing chiral side chain azobenzene monomer is synthesized by above reaction, the fluorine-containing chiral side chain azobenzene monomer includes a chiral molecular structure. A chiral molecule has a specific spatial structure, and is capable of occurring a chiral recognition interaction with the small molecule drugs, thereby increasing a desorption capacity of the small molecule drugs. An interaction between the small molecule drugs and the chiral molecule is weak, and thus the small molecule drugs are easily desorbed from materials by applying pressure or soaking in a solution. An adsorption position of the small molecule drugs in the materials can be limited by the spatial structure of the chiral molecule, thereby making the small molecule drugs easier to be desorbed. At the same time, the fluorine-containing chiral side chain azobenzene monomer also contains fluorine atoms, the fluorine atoms have high electronegativity, and chemical bond energy formed with other atoms is large, reducing an interaction force on a surface of prepared materials, and reducing a surface energy of the materials, thereby helping to weaken an interaction between the materials and the small molecule drugs, and increasing the desorption capacity of the small molecule drugs.

In the above process of the method for preparing the modified silica gel, the liquid silica gel is modified by the fluorine-containing chiral side chain azobenzene monomer, and the sampling material is obtained by the vacuum freeze-drying. In a process of the vacuum freeze-drying, water molecules in the liquid silica gel form ice crystals during freezing, and then the ice crystals are converted into water vapor by sublimation under a vacuum environment, therefore the water molecules in the liquid silica gel are removed, and a sampling material with porous structure is obtained. Pores are capable of providing larger surface area and more adsorption sites, thereby increasing an adsorption effect. The small molecule drugs are diffused into the pores and interacted with a surface of porous materials to achieve the adsorption effect.

In one embodiment, in the step (1), a dosage ratio of the pyridine, the 2-methyl-1-butanol and the p-toluenesulfonyl chloride is 80 to 90 mL : 8.5 to 8.7 g : 37.9 to 38.7 g, a cooling temperature is in a range of from 0 °C to 1°C, a stirring time of the stirring after adding the p-toluenesulfonyl chloride is in a range of from 0 hour to 1 hour, a storage time of storing the three-necked flask in the refrigerator is in a range of from 10 hours to 12 hours, and a stirring time of the stirring after adding the distilled water is in a range of from 2 hours to3 hours.

In one embodiment, in the step (2), during a process of obtaining the polymer 2, a dosage ratio of the 4-(4-hydroxybenzene) azobenzoic acid, the sodium bicarbonate, the dimethylacetamide and the polymer 1 is 2.3 to 2.5 g : 0.8 to 1.0 g : 25 to 30 mL : 2.8 to 3.2 g, a temperature of the heating is in a range of from 90°C to 100°C, the decreasing temperature is in a range of from 60°C to 70°C, the increasing temperature is in a range of from 80°C to 90°C, and a stirring time is in a range of from 24 hours to 30 hours.

In one embodiment, in the step (2), during a process of obtaining the polymer 3, a dosage ratio of the polymer 2, the potassium carbonate powder, the potassium iodide, the 6-chloro-1-hexanol and the dimethylamide is 1.0 to 1.3 g : 0.41 to 0.56 g : 0.007 to 0.008 g : 0.4 to 0.6 g : 25 to 35 mL, a temperature of the heating is in a range of from 110°C to 130°C, and a reacting time is in a range of from 70 hours to 75 hours.

In one embodiment, in the step (3), a dosage ratio of the polymer 3, the dichloromethane, the 4-dimethylaminopyridine and the methacrylic acid is 0.98 to 1.21 g : 26 to 32 mL : 0.04 to 0.05 g : 0.8 to 0.9 mL, during a process of obtaining the polymer 4, the increasing temperature is in a range of from 30°C to 35°C, and a reacting time is in a range of from 70 hours to 72 hours; during a process of obtaining the fluorine-containing chiral side chain azobenzene monomer, a temperature of the heating is in a range of from 30°C to 40°C, a reacting time is in a range of from 3 hours to 5hours, and a temperature of the heating after the reaction is completed is in a range of from 100°C to 120°C.

In one embodiment, in the step (4), a dosage ratio of the fluorine-containing chiral side chain azobenzene monomer and the liquid silica gel is 3 to 5 g : 30 to 42 mL.

In one embodiment, a sampling material of the continuous network skeleton structure is the melamine foam. Experiments prove that using the melamine foam as the sampling material, under a premise of ensuring efficient absorption, compared with the same type of foam material, the melamine foam enables significant low residual extrusion, which has unexpected technical effects. This may be due to a special polymer chain structure of the melamine foam and a honeycomb network structure formed by foaming. More than 99% of dirt and molecules can be absorbed powerfully and effectively, substances greater than the small molecule drugs and similar to cannabis small molecule drugs are priority adsorbed, hindering an absorption of the cannabis small molecule drugs, thereby effectively improving a recovery rate of effective substances.

The present invention also provides a use of the sampling material in sampling of small molecule drugs, including: using the sampling material to adsorb a liquid sample containing the small molecule drug molecules, and then squeezing the liquid sample out for testing.

The present invention also provides a highly efficient small molecule drug detection device, including:
a fluid sample collection element, including a liquid absorption element;
a desorption structure, contacted with the liquid absorption element to release a liquid sample; and including but not limited to an extrusion structure, squeezing the liquid absorption element; and
a test element, testing the liquid sample released by the liquid absorbent element.

The liquid absorption element adopts the low residue sampling material described above, including the continuous network skeleton structure, the density is in the range of from 8.3 kg/m³ to 9.3 kg/m³, the opening rate is greater than 99%, and the skeleton diameter is in the range of from 5 µm to 10 µm. The liquid absorption element preferably adopts the melamine foam, or the fluorine-containing chiral side chain azobenzene monomer modified liquid silica gel, thereby effectively improving a sample recovery rate.

Beneficial effects of the invention are as follows. A recovery rate of the small molecule drugs (BUP, 6MAM, PCP, FEN, BZO, THC (PARENT and METABOLITE)) is high, thereby effectively improving a detection rate.

The fluorine-containing chiral side chain azobenzene monomer includes the chiral molecular structure, and the chiral molecular structure is capable of occurring the chiral recognition interaction with the small molecule drugs, thereby increasing the desorption capacity of the small molecule drugs. Furthermore, the adsorption position of the small molecule drugs in the materials can be limited by the spatial structure of the chiral molecule, thereby making the small molecule drugs easier to be desorbed. At the same time, the fluorine atoms of the fluorine-containing chiral side chain azobenzene monomer have high electronegativity, thereby reducing the surface energy of the materials, weakening the interaction between the material and the small molecule drugs, further increasing the desorption capacity of the small molecule drugs and reducing the residual rate of the small molecule drugs in the sampling material.

### BRIEF DESCRIPTION OF DRAWINGS

FIG. 1 is an SEM image of melamine foam of the invention;
FIG. 2 is the SEM image of the melamine foam of the invention;
FIG. 3 is an SEM image of pva sponge;
FIG. 4 is the SEM image of the pva sponge;
FIG. 5 is an SEM image of a fiber sponge head;
FIG. 6 is the SEM image of the fiber sponge head;
FIG. 7 is a detection device for detection according to an embodiment of the invention.

### DESCRIPTION OF EMBODIMENTS

The present invention is further described below in conjunction with embodiments.

### Example 1:

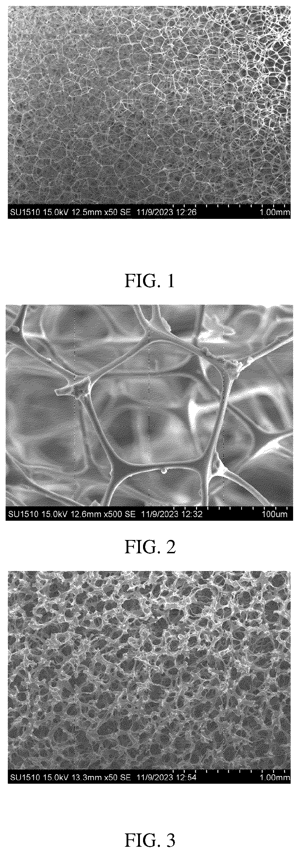

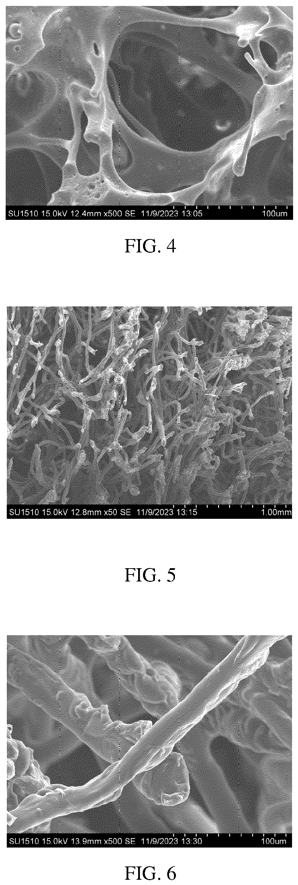

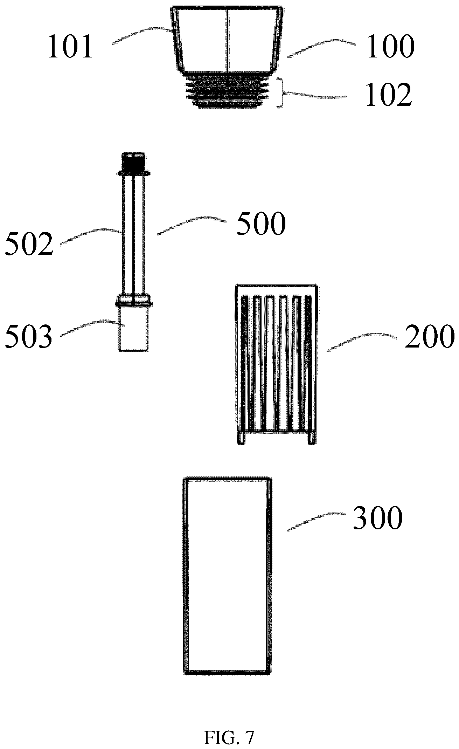

In the example, a melamine resin (market purchase, the density is 8.5 kg/m³, the opening rate is more than 99%, and the skeleton diameter is 5µm to 10 µm, as shown in FIG. 1 and FIG. 2), a pva sponge head (the density is 11.6g/cm³, as shown in FIG. 3 and FIG. 4) and a fiber sponge head (U.S. porex, about 20 µm in diameter, non-network skeleton as shown in FIG. 5 and FIG. 6) are used to construct an absorption element respectively, to form a detection device described in CN201821145987.6 patent. Specifically, the detection device includes a cavity 300 for accommodating a test element carrier and a test element carrier 200 (as shown in FIG. 7). A fluid collection element includes an absorbent element 503 for absorbing the fluid sample and a gripping part 100 with a flexible sheet structure 102. The gripping part 100 includes a hand-held part 101 and a plurality of ring-shaped flexible sheet structures 102 connected to the absorption element 503 by a connecting rod 502, thereby forming the fluid collection element. The absorption element 503 is put into a person's mouth (about 20 minutes) for a saliva collection. A collection element absorbed with a saliva sample is inserted into the cavity 300 for accommodating the test element carrier through an open of the cavity 300 for accommodating the test element carrier, with entry or insertion of the collection element, a liquid absorbing element enters a receiving cavity; and at this time, as a flexible sheet layer of an inner plug body gradually enters the opening of the cavity 300, cooperating with an inner wall, the absorption element 503 is compressed in an extrusion cavity, thereby releasing a liquid sample and testing an analyzed substance.

Product strips used to detect the analyzed substance are as follows:
1) BUP: 5 ng/ml colloidal gold test strip.
2) 6MAM: 10 ng/ml colloidal gold test strip.
3) PCP: 10 ng/ml colloidal gold test strip.
4) FEN: 10 ng/ml colloidal gold test strip.
5) BZO: 10 ng/ml colloidal gold test strip.
6) THC (PARENT): 40 ng/ml colloidal gold test strip.
7) THC (Metabolite): 12 ng/ml colloidal gold test strip.

Quality control materials:
1) BUP: 7.5 ng/ml (+50% cutoff), 15 ng/ml (3X cutoff).
2) 6MAM: 15 ng/ml (+50% cutoff), 30 ng/ml (3X cutoff).
3) PCP: 15 ng/ml (+50% cutoff), 30 ng/ml (3X cutoff).
4) FEN: 15 ng/ml (+50% cutoff), 30 ng/ml (3X cutoff).
5) BZO: 15 ng/ml (+50% cutoff), 30 ng/ml (3X cutoff).
6) THC (PARENT): 60 ng/ml (+50% cutoff), 120 ng/ml (3X cutoff).
7) THC (Metabolite): 18 ng/ml (+50% cutoff), 36 ng/ml (3X cutoff).
8) Fresh non-drug saliva.

An operation method is described as follows:
DEVICE E: all kinds of SWAB are immersed in a solution for about 20 minutes, after 20 minutes, the solution is extruded, and samples (original standard without SWAB soaking and various standards after various SWABs soaking) are added by a pipette gun for comparative testing.
MIDSTREAM: comparison: removing the SWAB and directly adding samples with the pipette gun for testing; test group: assembling the SWAB in a DMISTREAM mold, and adding samples for testing, to compare.

**Table 1 BUP test results**

| Fresh non-drug saliva | | | | |
|---|---|---|---|---|
| product | adding samples with the pipette gun (comparison) | melamine resin | pva sponge head | fiber sponge head |
| DEVICE E | G9 | G9 | G9 | G9 |
| MIDSTREAM | G9 | G9 | G9 | G9 |
| LC/MS (ng/ml) | 0 | 0 | 0 | 0 |

| 7.5 ng/ml (+50% cutoff) | | | | |
|---|---|---|---|---|
| product | adding samples with the pipette gun (comparison) | melamine resin | pva sponge head | fiber sponge head |
| DEVICE E | G3.5 | G3.5 | G4.5 | G4.5 |
| MIDSTREAM | G3.5 | G3.5 | G4.5 | G4.5 |
| LC/MS (ng/ml) | 7.35 | 7.36 | 5.02 | 4.88 |
| recovery rate | 98.00% | 98.13% | 66.93% | 65.07% |

| 15 ng/ml (3X cutoff) | | | | |
|---|---|---|---|---|
| product | adding samples with the pipette gun (comparison) | melamine resin | pva sponge head | fiber sponge head |
| DEVICE E | G2 | G2 | G3 | G3 |
| MIDSTREAM | G2 | G2 | G3 | G3 |
| LC/MS (ng/ml) | 15.18 | 15.02 | 10.65 | 12.63 |
| recovery rate | 100.00% | 100.00% | 71.00% | 84.20% |

**Table 2 6MAM test results**

| Fresh non-drug saliva | | | | |
|---|---|---|---|---|
| product | adding samples with the pipette gun (comparison) | melamine resin | pva sponge head | fiber sponge head |
| DEVICE E | G9.5 | G9.5 | G9.5 | G9.5 |
| MIDSTREAM | G9.5 | G9.5 | G9.5 | G9.5 |
| LC/MS (ng/ml) | 0 | 0 | 0 | 0 |

| 15 ng/ml (+50% cutoff) | | | | |
|---|---|---|---|---|
| product | adding samples with the pipette gun (comparison) | melamine resin | pva sponge head | fiber sponge head |
| DEVICE E | G3.5 | G3.5 | G5 | G4.5-5 |
| MIDSTREAM | G3.5 | G3.5 | G5 | G4.5-5 |
| LC/MS (ng/ml) | 15.58 | 15.65 | 11.32 | 10.52 |
| recovery rate | 100.00% | 100.00% | 75.47% | 70.13% |

| 30 ng/ml (3X cutoff) | | | | |
|---|---|---|---|---|
| product | adding samples with the pipette gun (comparison) | melamine resin | pva sponge head | fiber sponge head |
| DEVICE E | G2 | G2 | G3 | G3 |
| MIDSTREAM | G2 | G2 | G3 | G3 |
| LC/MS (ng/ml) | 33.15 | 34.17 | 19.56 | 21.34 |
| recovery rate | 100.00% | 100.00% | 65.20% | 71.13% |

**Table 3 PCP test results**

| Fresh non-drug saliva | | | | |
|---|---|---|---|---|
| product | adding samples with the pipette gun (comparison) | melamine resin | pva sponge head | fiber sponge head |
| DEVICE E | G9.5 | G9.5 | G9.5 | G9.5 |
| MIDSTREAM | G9.5 | G9.5 | G9.5 | G9.5 |
| LC/MS (ng/ml) | 0 | 0 | 0 | 0 |

| 15 ng/ml (+50% cutoff) | | | | |
|---|---|---|---|---|
| product | adding samples with the pipette gun (comparison) | melamine resin | pva sponge head | fiber sponge head |
| DEVICE E | G3 | G3 | G4 | G4 |
| MIDSTREAM | G3 | G3 | G4 | G4 |
| LC/MS (ng/ml) | 13.52 | 14.15 | 12.13 | 11.89 |
| recovery rate | 90.13% | 94.33% | 80.87% | 79.27% |

| 30 ng/ml (3X cutoff) | | | | |
|---|---|---|---|---|
| product | adding samples with the pipette gun (comparison) | melamine resin | pva sponge head | fiber sponge head |
| DEVICE E | G2 | G2 | G3 | G3 |
| MIDSTREAM | G2 | G2 | G3 | G3 |
| LC/MS (ng/ml) | 29.12 | 30.15 | 19.51 | 18.84 |
| recovery rate | 97.07% | 100.50% | 65.03% | 62.80% |

**Table 4 FEN test results**

| Fresh non-drug saliva | | | | |
|---|---|---|---|---|
| product | adding samples with the pipette gun (comparison) | melamine resin | pva sponge head | fiber sponge head |
| DEVICE E | G9.5 | G9.5 | G9.5 | G9.5 |
| MIDSTREAM | G9.5 | G9.5 | G9.5 | G9.5 |
| LC/MS (ng/ml) | 0 | 0 | 0 | 0 |

| 15 ng/ml (+50% cutoff) | | | | |
|---|---|---|---|---|
| product | adding samples with the pipette gun (comparison) | melamine resin | pva sponge head | fiber sponge head |
| DEVICE E | G3.5 | G3.5 | G5 | G5 |
| MIDSTREAM | G3.5 | G3.5 | G5 | G5 |
| LC/MS (ng/ml) | 14.68 | 15.65 | 12.82 | 11.57 |
| recovery rate | 97.87% | 100.00% | 85.47% | 77.13% |

| 30 ng/ml (3X cutoff) | | | | |
|---|---|---|---|---|
| product | adding samples with the pipette gun (comparison) | melamine resin | pva sponge head | fiber sponge head |
| DEVICE E | G2 | G2 | G3.5 | G3.5 |
| MIDSTREAM | G2 | G2 | G3.5 | G3.5 |
| LC/MS (ng/ml) | 31.15 | 30.17 | 22.56 | 19.34 |
| recovery rate | 100.00% | 100.00% | 75.20% | 64.47% |

**Table 5 BZO test results**

| Fresh non-drug saliva | | | | |
|---|---|---|---|---|
| product | adding samples with the pipette gun (comparison) | melamine resin | pva sponge head | fiber sponge head |
| DEVICE E | G9 | G9 | G9 | G9 |
| MIDSTREAM | G9 | G9 | G9 | G9 |
| LC/MS (ng/ml) | 0 | 0 | 0 | 0 |

| 15 ng/ml (+50% cutoff) | | | | |
|---|---|---|---|---|
| product | adding samples with the pipette gun (comparison) | melamine resin | pva sponge head | fiber sponge head |
| DEVICE E | G3.5 | G3.5 | G4.5 | G4.5-5 |
| MIDSTREAM | G3.5 | G3.5 | G5 | G5 |
| LC/MS (ng/ml) | 14.58 | 13.65 | 10.32 | 9.52 |
| recovery rate | 97.20% | 91.00% | 68.80% | 63.47% |

| 30 ng/ml (3X cutoff) | | | | |
|---|---|---|---|---|
| product | adding samples with the pipette gun (comparison) | melamine resin | pva sponge head | fiber sponge head |
| DEVICE E | G2 | G2 | G3.5 | G3.5 |
| MIDSTREAM | G2 | G2 | G3.5 | G3.5 |
| LC/MS (ng/ml) | 31.95 | 32.13 | 20.96 | 22.11 |
| recovery rate | 100.00% | 100.00% | 69.87% | 73.70% |

**Table 6 THC (parent) test results**

| Fresh non-drug saliva | | | | |
|---|---|---|---|---|
| product | adding samples with the pipette gun (comparison) | melamine resin | pva sponge head | fiber sponge head |
| DEVICE E | G9 | G9 | G9 | G9 |
| MIDSTREAM | G9 | G9 | G9 | G9 |
| LC/MS (ng/ml) | 0 | 0 | 0 | 0 |

| 60 ng/ml (+50% cutoff) | | | | |
|---|---|---|---|---|
| product | adding samples with the pipette gun (comparison) | melamine resin | pva sponge head | fiber sponge head |
| DEVICE E | G3.5 | G3.5 | G8 | G8 |
| MIDSTREAM | G3.5 | G3.5 | G8 | G8 |
| LC/MS (ng/ml) | 53.58 | 52.65 | 10.52 | 9.52 |
| recovery rate | 89.30% | 87.75% | 17.53% | 15.87% |

| 120 ng/ml (3X cutoff) | | | | |
|---|---|---|---|---|
| product | adding samples with the pipette gun (comparison) | melamine resin | pva sponge head | fiber sponge head |
| DEVICE E | G2 | G2 | G7 | G7 |
| MIDSTREAM | G2 | G2 | G7 | G7 |
| LC/MS (ng/ml) | 109.25 | 103.17 | 29.56 | 30.34 |
| recovery rate | 91.04% | 85.98% | 24.63% | 25.28% |

**Table 7 THC (metabolite) test results**

| Fresh non-drug saliva | | | | |
|---|---|---|---|---|
| product | adding samples with the pipette gun (comparison) | melamine resin | pva sponge head | fiber sponge head |
| DEVICE E | G9.5 | G9.5 | G9.5 | G9.5 |
| MIDSTREAM | G9.5 | G9.5 | G9.5 | G9.5 |
| LC/MS (ng/ml) | 0 | 0 | 0 | 0 |

| 18 ng/ml (+50% cutoff) | | | | |
|---|---|---|---|---|
| product | adding samples with the pipette gun (comparison) | melamine resin | pva sponge head | fiber sponge head |
| DEVICE E | G3 | G3 | G6 | G6 |
| MIDSTREAM | G3 | G3 | G6 | G6 |
| LC/MS(ng/ml) | 16.58 | 14.28 | 5.32 | 4.52 |
| recovery rate | 92.11% | 79.33% | 29.56% | 25.11% |

| 36 ng/ml (3X cutoff) | | | | |
|---|---|---|---|---|
| product | adding samples with the pipette gun (comparison) | melamine resin | pva sponge head | fiber sponge head |
| DEVICE E | G2 | G2 | G5 | G5 |
| MIDSTREAM | G2 | G2 | G5 | G5 |
| LC/MS (ng/ml) | 30.15 | 30.07 | 6.56 | 7.34 |
| recovery rate | 83.75% | 83.53% | 18.22% | 20.39% |

In conclusion: the adsorption of BUP, 6MAM, PCP, FEN, BZO, THC (PARENT and METABOLITE) in the small molecule drugs can be effectively reduced by melamine foam materials, and the recovery rate is more than 79%, thereby effectively improving the detection rate.

### Example 2:

A superhydrophobic modified melamine foam of the example is prepared by the following steps:
(1) dispersing one part by weight of sodium persulfate initiator in 1000 parts by weight of deionized water to obtain a solution A containing sodium persulfate;
(2) dispersing 30 parts by weight of styrene, 22 parts by weight of divinylbenzene, 16 parts by weight of emulsifier Span80 and 4.3 parts by weight of stearic acid in 1001 parts by weight of the solution A, to obtain a mixed solution B;
(3) dispersing the mixed solution B in anhydrous ethanol, to obtain a hydrophobic modified emulsion;
(4) ultrasonically immersing 5 parts by weight of melamine foam in the hydrophobically modified emulsion for 64 minutes, and then reacting at 65°C for 8.5 hours, washing a product with anhydrous ethanol, and then blast drying at 50 °C for 10 h, to obtain the superhydrophobic modified melamine foam.

### Example 3:

A superhydrophobic modified melamine foam of the example is prepared by the following steps:
(1) dispersing one part by weight of sodium persulfate initiator in 1000 parts by weight of deionized water to obtain a solution A containing initiator;
(2) dispersing 21 parts by weight of methyl methacrylate, 20 parts by weight of N,N-methylenebisacrylamide, 10 parts by weight of emulsifier Tween80 and 8 parts by weight of stearic acid in 1001 parts by weight of the solution A., to obtain a mixed solution B;
(3) dispersing the mixed solution B in tetrahydrofuran to obtain a hydrophobically modified emulsion;
(4) ultrasonically immersing 5 parts by weight of melamine foam (market purchase, the density is 8.7 kg/m³, the opening rate is more than 99%, and the skeleton diameter is 5µm to 10 µm) in the hydrophobically modified emulsion for 30 minutes, and then reacting at 40°C for 24 hours, washing a product with anhydrous ethanol, and then blast drying at 100°C for 8 hours, to obtain the superhydrophobic modified melamine foam.

The superhydrophobic modified melamine foam obtained in Example 2 and Example 3 are used to adsorb buprenorphine with a known concentration, the small molecule drugs are desorbed by extrusion, a concentration of desorbed small molecule drugs is measured and a residual rate is calculated. The residual rate of a sample obtained in Example 2 is 1.3%, and the residual rate of a sample obtained in Example 3 is 1.8%.

### Example 4:

The continuous network skeleton structure of the sampling material is mainly composed of silica gel modified with the fluorine-containing chiral side chain azobenzene monomer, and a method for preparing the silica gel modified with the fluorine-containing chiral side chain azobenzene monomer includes the following steps:
step (1), adding 85g of pyridine, 8.6g of 2-methyl-1-butanol into a three-necked flask, and then placing the three-necked flask in an ice water bath; cooling to 0°C, adding 38g of p-toluenesulfonyl chloride, and controlling a temperature of the three-necked flask and stirring for 1 hour, and then storing the three-necked flask in a refrigerator for 12 hours; removing the three-necked flask and placing the three-necked flask in the ice water bath, and adding distilled water and stirring for 3 hours; after a reaction is completed, adding ether for extraction, collecting organic phases and washing with hydrochloric acid and water; and then adding anhydrous sodium sulfate for drying, and filtering to obtain a polymer 1;
step (2), adding 2.4 g of 4-(4-hydroxyphenyl)azobenzoic acid, 0.9g of sodium bicarbonate and 27 mL of dimethylacetamide into a three-necked flask, heating to 100°C, and stirring until solids are completely dissolved, and then decreasing temperature to 60°C; adding 3.0g of the polymer 1 dropwise into the three-necked flask, increasing temperature to 90°C, and stirring for 24 hours, after stirring, cooling, and then adding distilled water and filtering to obtain a polymer 2; adding 1.2 g of the polymer 2, 0.5g of potassium carbonate powder and 0.0075g of potassium iodide into a three-necked flask in sequence, and then adding 30 mL of dimethylformamide to obtain a reaction solution; adding 6-chloro-1-hexanol dropwise into the reaction solution under stirring, heating to 120°C, and reacting for 72 hours, after the reaction is completed, cooling, adding distilled water to the three-port flask, pumping and filtering to obtain a polymer 3;
step (3), adding 1.03g of the polymer 3, 30mL of dichloromethane, 0.048g of 4-dimethylaminopyridine and 0.85mL of methacrylic acid into a single flask, stirring until completely dissolved; adding a mixed solution of dicyclohexylcarbodiimide and dichloromethane, increasing temperature to 30°C and reacting for 72 hours, after the reaction is completed, filtrating, and rotary evaporating to obtain a polymer 4; dissolving the polymer 4 in hydrofluoric acid solution, then adding hydrogen peroxide, heating to 33°C, stirring, and reacting for 4 hours, after the reaction is completed, heating to 120°C, and suction filtering to obtain a fluorine-containing chiral side chain azobenzene monomer;
step (4), dissolving 4g of the fluorine-containing chiral side chain azobenzene monomer in cyclopentanone, stirring until completely dissolved to obtain a mixed solution, and then adding the mixed solution into 38mL of liquid silica gel, stirring to mix evenly, vacuum freeze-drying, to obtain a sampling material with a cross-linked network structure. The sampling material with the cross-linked network structure has a density of 8.3kg/m³, an open porosity of more than 99%, and a skeleton diameter of 5µm to 10µm.

### Example 5:

The continuous network skeleton structure in the sampling material is mainly composed of silica gel modified with the fluorine-containing chiral side chain azobenzene monomer, and a method for preparing the silica gel modified with the fluorine-containing chiral side chain azobenzene monomer includes the following steps:
step (1), adding 82g of pyridine, 8.5g of 2-methyl-1-butanol into a three-necked flask, and then placing the three-necked flask in an ice water bath; cooling to 0°C, adding 38.1g of p-toluenesulfonyl chloride, and controlling a temperature of the three-necked flask and stirring for 1 hour, and then storing the three-necked flask in a refrigerator for 12 hours; removing the three-necked flask and placing the three-necked flask in the ice water bath, and adding distilled water and stirring for 3 hours; after a reaction is completed, adding ether for extraction, collecting organic phases and washing with hydrochloric acid and water; and then adding anhydrous sodium sulfate for drying, and filtering to obtain a polymer 1;
step (2), adding 2.3g of 4-(4-hydroxyphenyl)azobenzoic acid, 0.8g of sodium bicarbonate and 25 mL of dimethylacetamide into a three-necked flask, heating to 100°C, and stirring until solids are completely dissolved, and then decreasing temperature to 60°C; adding 2.9g of the polymer 1 dropwise into the three-necked flask, increasing temperature to 90°C, and stirring for 24 hours, after stirring, cooling, and then adding distilled water and filtering to obtain a polymer 2; adding 1.1g of the polymer 2, 0.45g of potassium carbonate powder and 0.0071g of potassium iodide into a three-necked flask in sequence, and then adding 28mL of dimethylformamide to obtain a reaction solution; and then adding 6-chloro-1-hexanol dropwise into the reaction solution under stirring, heating to 120°C, and reacting for 72 hours, after the reaction is completed, cooling, adding distilled water to the three-port flask, pumping and filtering to obtain a polymer 3;
step (3), adding 0.99g of the polymer 3, 27mL of dichloromethane, 0.042g of 4-dimethylaminopyridine and 0.81mL of methacrylic acid into a single flask, stirring until completely dissolved; and then adding a mixed solution of dicyclohexylcarbodiimide and dichloromethane, increasing temperature to 30°C and reacting for 72 hours, after the reaction is completed, filtrating, and rotary evaporating to obtain a polymer 4; dissolving the polymer 4 in hydrofluoric acid solution, then adding hydrogen peroxide, heating to 33°C, stirring, and reacting for 4 hours, after the reaction is completed, heating to 120°C, and suction filtering to obtain a fluorine-containing chiral side chain azobenzene monomer;
step (4), dissolving 3g of the fluorine-containing chiral side chain azobenzene monomer in cyclopentanone, stirring until completely dissolved to obtain a mixed solution, and then adding the mixed solution into 35mL of liquid silica gel, stirring to mix evenly, vacuum freeze-drying, to obtain a sampling material with a cross-linked network structure. The sampling material with the cross-linked network structure has a density of 9.3kg/m³, an open porosity of more than 99%, and a skeleton diameter of 5 µm to 10µm.

### Example 6:

The continuous network skeleton structure of the sampling material is mainly composed of silica gel modified with the fluorine-containing chiral side chain azobenzene monomer, and a method for preparing the silica gel modified with the fluorine-containing chiral side chain azobenzene monomer includes the following steps:
step (1), adding 87g of pyridine, 8.7g of 2-methyl-1-butanol into a three-necked flask, and then placing the three-necked flask in an ice water bath; cooling to 0°C, adding 38.6g of p-toluenesulfonyl chloride, and controlling a temperature of the three-necked flask and stirring for 1 hour, and then storing the three-necked flask in a refrigerator for 12 hours; removing the three-necked flask and placing the three-necked flask in the ice water bath, and adding distilled water and stirring for 3 hours; after a reaction is completed, adding ether for extraction, collecting organic phases and washing with hydrochloric acid and water; and then adding anhydrous sodium sulfate for drying, and filtering to obtain a polymer 1;
step (2), adding 2.5g of 4-(4-hydroxyphenyl)azobenzoic acid, 1.0g of sodium bicarbonate and 30 mL of dimethylacetamide into a three-necked flask, heating to 100°C, and stirring until solids are completely dissolved, and then decreasing temperature to 60°C; adding 3.1g of the polymer 1 dropwise into the three-necked flask, increasing temperature to 90°C, and stirring for 24 hours, after stirring, cooling, and then adding distilled water and filtering to obtain a polymer 2; adding 1.25g of the polymer 2, 0.54g of potassium carbonate powder and 0.0078g of potassium iodide into a three-necked flask in sequence, and then adding 28mL of dimethylformamideto obtain a reaction solution; then adding 6-chloro-1-hexanol dropwise into the reaction solution under stirring, heating to 120°C, and reacting for 72 hours, after the reaction is completed, cooling, adding distilled water to the three-port flask, pumping and filtering to obtain a polymer 3;
step (3), adding 1.18g of the polymer 3, 31mL of dichloromethane, 0.045g of 4-dimethylaminopyridine and 0.87mL of methacrylic acid into a single flask, stirring until completely dissolved; adding a mixed solution of dicyclohexylcarbodiimide and dichloromethane, increasing temperature to 30°C and reacting for 72 hours, after the reaction is completed, filtrating, and rotary evaporating to obtain a polymer 4; dissolving the polymer 4 in hydrofluoric acid solution, then adding hydrogen peroxide, heating to 33°C, stirring, and reacting for 4 hours, after the reaction is completed, heating to 120°C, and suction filtering to obtain a fluorine-containing chiral side chain azobenzene monomer;
step (4), dissolving 5g of the fluorine-containing chiral side chain azobenzene monomer in cyclopentanone, stirring until completely dissolved to obtain a mixed solution, and then adding the mixed solution into 41mL of liquid silica gel, stirring to mix evenly, vacuum freeze-drying, to obtain a sampling material with a cross-linked network structure. The sampling material with the cross-linked network structure has a density of 8.9kg/m³, an open porosity of more than 99%, and a skeleton diameter of 5µm to 10µm.

### Comparative Example 1:

During preparing a sampling material, the fluorine-containing chiral side chain azobenzene monomer is not used, and the liquid silica gel is directly used. Other conditions are the same as in Example 4.

The superhydrophobic modified melamine foam obtained in Example 4, Example 5, Example 6 and Comparative Example 1 are used to adsorb 6-MAM with a known concentration, the 6-MAM are desorbed by squeezing or soaking in liquid, a concentration of desorbed 6-MAM is measured and a residual rate is calculated. Test results are shown in the following table:

| | Example 4 | Example 5 | Example 6 | Comparative Example 1 |
|---|---|---|---|---|
| residual rate/% | 23.3 | 24.1 | 26.8 | 35.6 |

From the test results in the table, it can be seen that the sampling materials prepared in Examples 2-4 of the present invention have a relatively low residual rate of the small molecule drugs compared to existing products (PVA sponge head, fiber sponge head (Porex, USA)). From a comparison between Comparative Example 1 and Examples 2-4, it can be seen that the residual rate of the small molecule drugs can be effectively reduced by an addition of the fluorine-containing chiral side chain azobenzene monomer.

## Claims

1. A low residue sampling material for small molecule drug detection, comprising: a continuous network skeleton structure, wherein:
a density is in a range of from 8.3 kg/m³ to 9.3 kg/m³, an opening rate is greater than 99%, and a skeleton diameter is in a range of from 5 µm to 10 µm.

2. A use of the low residue sampling material of claim 1 in a sampling of small molecule drugs, comprising:
using the low residue sampling material to adsorb liquid samples comprising small molecule drugs; and then squeezing out the liquid samples for testing.

3. The use of the low residue sampling material in the sampling of small molecule drugs of claim 2, wherein materials with the continuous network skeleton structure comprise polyurethane foam, melamine foam, activated carbon, polydimethylsiloxane sponge, modified silica gel, polyester fibers and polyester films.

4. The use of the low residue sampling material in the sampling of small molecule drugs of claim 3, wherein a method for preparing the melamine foam comprises steps of: impregnating the melamine foam by an hydrophobic modification emulsion; and then, performing an emulsion polymerization under a heating condition, and in situ growing nanospheres on a melamine foam skeleton; and finally, washing and drying to obtain superhydrophobic modified melamine foam.

5. The use of the low residue sampling material in the sampling of small molecule drugs of claim 4, wherein a method for preparing a hydrophobic modification emulsion comprises steps of:
dispersing an initiator in deionized water, and adding a modified monomer, a crosslinker, an emulsifier and a low surface energy modifier, to obtain a mixed solution; and
dispersing the mixed solution in the solvent to obtain the hydrophobic modification emulsion,
wherein, by a weight part, in the hydrophobic modified emulsion, the modified monomer is in a range of from 21 parts to 30 parts, the initiator is 1 part, the crosslinker is in a range of from 20 parts to 22 parts, the emulsifier is in a range of from 10 parts to 16 parts, and the low surface energy modifier is in a range of from 4.3 parts to 8 parts.

6. The use of the low residue sampling material in the sampling of small molecule drugs of claim 5, wherein the modified monomer is one of styrene, acrylic acid, and methyl methacrylate; and the low surface energy modifier is stearic acid or dimethicone.

7. The use of the low residue sampling material in the sampling of small molecule drugs of claim 5, wherein the initiator is sodium persulfate or ammonium persulfate; and the crosslinker is one of divinylbenzene, N,N-methylenebisacrylamide, trimethylolpropane triacrylate, ethylene glycol dimethacrylate, and dipentaerythritol pentaacrylate.

8. The use of the low residue sampling material in the sampling of small molecule drugs of claim 5, wherein the emulsifier is one of Span80, Tween80, hexadecyltrimethylammonium bromide, sodium dodecyl sulfate, and ethylene glycol fatty acid ester.

9. The use of the low residue sampling material in the sampling of small molecule drugs of claim 3, wherein a method for preparing the modified silica gel comprises steps of:
step (1), adding pyridine and 2-methyl-1-butanol into a three-necked flask, and then placing the three-necked flask in an ice water bath; after cooling, adding p-toluenesulfonyl chloride, and controlling a temperature of the three-necked flask and stirring, and then storing the three-necked flask in a refrigerator; removing the three-necked flask and placing the three-necked flask in the ice water bath, and adding distilled water and stirring; after a reaction is completed, adding ether for extraction, collecting organic phases and washing with hydrochloric acid and water; and then adding anhydrous sodium sulfate for drying, and filtering to obtain a polymer 1;
step (2), adding 4-(4-hydroxyphenyl)azobenzoic acid, sodium bicarbonate and dimethylacetamide into a three-necked flask, heating and stirring until solids are completely dissolved, and then decreasing temperature; adding the polymer 1 into the three-necked flask, increasing temperature and stirring; and after stirring, cooling, adding distilled water and suction filtering to obtain a polymer 2; and
adding the polymer 2, potassium carbonate powder and potassium iodide into a three-necked flask in sequence, and then adding dimethyl methacrylate to obtain a reaction solution; adding 6-chloro-1-hexanol dropwise into the reaction solution under stirring, heating and reacting; and after reaction is completed, cooling, adding distilled water to the three-port flask, pumping and suction filtering to obtain a polymer 3;
step (3), adding the polymer 3, dichloromethane, 4-dimethylaminopyridine and methacrylic acid into a single flask, stirring until completely dissolved; adding a mixed solution of dicyclohexylcarbodiimide and dichloromethane, increasing temperature and reacting; and after a reaction is completed, filtrating, and rotary evaporating to obtain a polymer 4; and dissolving the polymer 4 in a hydrofluoric acid solution, adding hydrogen peroxide, heating, stirring and reacting; and after a reaction is completed, heating and suction filtering to obtain a fluorine-containing chiral side chain azobenzene monomer; and
step (4), dissolving the fluorine-containing chiral side chain azobenzene monomer in cyclopentanone, stirring until completely dissolved to obtain a mixed solution, and then adding the mixed solution into a liquid silica gel, stirring to mix evenly, and vacuum freeze-drying, to obtain the sampling material.

10. The use of the low residue sampling material in the sampling of small molecule drugs of claim 9, wherein in the step (1), a dosage ratio of the pyridine, the 2-methyl-1-butanol and the p-toluenesulfonyl chloride is 80 to 90 mL : 8.5 to 8.7 g : 37.9 to 38.7 g, a cooling temperature is in a range of from 0°C to 1°C, a stirring time of the stirring after adding the p-toluenesulfonyl chloride is in a range of from 0 hour to 1 hour, a storage time of storing the first three-necked flask in the refrigerator is in a range of from 10 hours to 12 hours, and a stirring time of the stirring after adding the distilled water is in a range of from 2 hours to 3 hours.

11. The use of the low residue sampling material in the sampling of small molecule drugs of claim 9, wherein in the step (2), during a process of obtaining the polymer 2, a dosage ratio of the 4-(4-hydroxybenzene) azobenzoic acid, the sodium bicarbonate, the dimethylacetamide and the polymer 1 is 2.3 to 2.5 g : 0.8 to 1.0 g : 25 to 30 mL : 2.8 to 3.2 g, a temperature of the heating is in a range of from 90°C to 100°C, the decreasing temperature is in a range of from 60°C to 70°C, the increasing temperature is in a range of from 80°C to 90°C, and a stirring time is in a range of from 24 hours to 30 hours.

12. A highly efficient small molecule drug detection device, comprising:
a fluid sample collection element, comprising a liquid absorption element;
a desorption structure, contacted with the liquid absorption element to release a liquid sample; and
a test element, configured to test the liquid sample released by the absorbent element,
wherein the liquid absorption element adopts the low-residue sampling material of claim 1.
